# EUROPÄISCHE PATENTSCHRIFT

(11) **EP 0 934 238 B1**
(45) Veröffentlichungstag und Bekanntmachung des Hinweises auf die Patenterteilung: **12.09.2001**
(21) Anmeldenummer: 97948761.8
(22) Anmeldetag: 17.10.1997
(51) Int. Cl.: C07C 27/12, C07C 29/50, C07C 45/33

(54) **VERFAHREN ZUR HERSTELLUNG VON OXIDATIONSPRODUKTEN DES CYCLOHEXANS IM GEGENSTROM**
PROCESS FOR PREPARING OXIDATION PRODUCTS FROM CYCLOHEXANE IN COUNTERFLOW
PROCEDE DE PREPARATION DE PRODUITS D'OXYDATION DE CYCLOHEXANE A CONTRE-COURANT

(30) Priorität: 18.10.1996 DE 19643154
(43) Veröffentlichungstag der Anmeldung: 11.08.1999
(73) Patentinhaber: BASF AKTIENGESELLSCHAFT, 67056 Ludwigshafen (DE)
(72) Erfinder: REHFINGER, Alwin, D-67112 Mutterstadt (DE); GANN, Martin, D-67251 Freinsheim (DE); MÄRKL, Robert, Randolph, NJ 07869 (US); SCHMITT, Rüdiger, D-67227 Frankenthal (DE)
(74) Vertreter: Kinzebach, Werner, Dr.
(86) Internationale Anmeldenummer: EP9705740
(87) Internationale Veröffentlichungsnummer: WO9817612

(56) Entgegenhaltungen:
- DE-A- 2 136 744
- DE-A- 3 328 771

## Beschreibung

Die vorliegende Erfindung betrifft ein kontinuierliches Verfahren zur Herstellung von Oxidationsprodukten des Cyclohexans durch katalytische Oxidation mit sauerstoffhaltigen Gasen in flüssiger Phase, wobei man die Gase in mindestens einer Reaktionszone im wesentlichen gleichmäßig mit flüssigem Cyclohexan in Kontakt bringt.

Verfahren zur Oxidation von Kohlenwasserstoffen mit molekularem Sauerstoff und insbesondere die Oxidation von Cyclohexan zu Cyclohexanol und Cyclohexanon sind nach dem Stand der Technik bekannt. So beschreibt die DE-A-21 36 744 sowie die US-A-3,957,876 ein Verfahren zur Herstellung von Cyclohexylhydroperoxid-haltigen Lösungen durch zonenweise Oxidation von Cyclohexan, wobei man ein Gemisch aus Cyclohexan und einem löslichen Kobaltkatalysator von oben nach unten und ein sauerstoffhaltiges Gas im Gegenstrom von unten nach oben durch eine Bodenkolonne strömen lässt. Diese umfasst am oberen Ende eine Sauerstoff-Aufbrauchzone und daran nach unten anschliessend Oxidations-Zonen, wobei letztere jeweils separat mit unterschiedlichen Sauerstoffmengen gespeist werden können. Das Verfahren zielt ausschließlich auf die Herstellung von Cyclohexylhydroperoxid, welches in einer Menge von 15 Gew.-%, bezogen auf die Gesamtmenge an Oxidationsprodukten, erhalten wird.

Die US-A-4,675,450 beschreibt ein zur DE-A-21 36 744 analoges Verfahren zur Herstellung von Cyclohexylhydroperoxid, wobei die Cyclohexanoxidation in Gegenwart eines löslichen Kobaltkatalysators sowie eines Phosphorsäureesters durchgeführt wird.

Die DE-A-12 87 575 beschreibt ein Verfahren zur Cyclohexanoxidation von flüssigem Cyclohexan in mehreren, unmittelbar aufeinanderfolgenden Oxidationsstufen, wobei in jede Oxidationsstufe sauerstoffhaltiges Gas eingeleitet wird. Diese Einleitung erfolgt so, daß die Geschwindigkeit der Sauerstoffzuführung der Geschwindigkeit des Sauerstoffverbrauchs in jeder Stufe im wesentlichen entspricht, wobei in die letzte Oxidationsstufe zusätzlich Inertgas geleitet wird. Somit kommt es zwangsläufig zu einer ungleichmäßigen Sauerstoffzuführung und -verteilung im Reaktionsgemisch, was zu einer Ausbeuteverringerung führt. Die Reaktionszone ist durch nach unten gebogene Bleche, die nicht den gesamten Querschnitt bedecken, in Kammern gegliedert. Die Einspeisung des Gases erfolgt unterhalb dieser Bleche in die abströmende Gasphase der nachfolgenden Oxidationsstufe, was bei einer als geeignet beschriebenen Reaktionsführung mit 'befluteten Böden' ebenfalls zu einer ungleichmäßigen Sauerstoffzuführung, zu einem ungleichmäßigen Fließen des Reaktionsgemisches und im ungünstigsten Fall zur Ausbildung von sauerstoffhaltigen Gasräumen unter den Böden führen kann, wodurch die Gefahr der Bildung eines zündfähigen Gemisches und einer Explosion erhöht wird.

Die DE-C-25 15 419, entsprechend der US-A-3,987,100, beschreibt ein Verfahren zur Herstellung von Cyclohexanon und Cyclohexanol durch Oxidation von Cyclohexan in einer im Gegenstrom betriebenen Bodenkolonne in Gegenwart eines löslichen, binären Katalysatorsystems mit 0,1 bis 5 ppm Kobalt und 0,02 bis 0,9 ppm Chrom. Dabei sind die einzelnen Böden z.B. als Lochbleche ausgeführt, durch die das sauerstoffhaltige Gas aufsteigen und durch das von oben herabfliessende Cyclohexan strömen kann.

In Analogie zur DE-A-12 87 575 kann auf einem Teil oder allen der Böden (mit Ausnahme einer Sauerstoffaufbrauchzone am Kolonnenkopf) zusätzlich sauerstoffhaltiges Gas eingeführt werden. Diese Einführung erfolgt wiederum so, daß praktisch der gesamte in jede Stufe eingeführte Sauerstoff in dieser Stufe auch verbraucht wird. Da zudem die Anzahl und/oder die Größe der Löcher in den Lochblechen vom Boden zum Kopf des Reaktors größer wird und da in die oberen Böden kein Sauerstoff eingespeist wird, treten bei diesem Verfahren, wie schon zuvor bei der DE-A-12 87 575 beschrieben, eine ungleichmäßige Sauerstoffzuführung, eine ungleichmäßige Sauerstoffverteilung im Reaktor und ein ungleichmäßiger Fluß des Reaktionsgemisches auf. Da zudem die freie Bodenfläche mit ca. 1,2 % sehr gering ist, kann es auch bei diesem Verfahren zur Ausbildung von Gasräumen unter den Böden mit der zuvor beschriebenen Explosionsgefahr kommen.

Keine der zuvor genannten Schriften beschreibt ein Verfahren zur Herstellung von Oxidationsprodukten des Cyclohexans, wobei das sauerstoffhaltige Gas direkt in die flüssige Phase eingeleitet wird und dort Blasen bildet, so daß keine zusammenhängende Gasphase mehr im Reaktor besteht, und bei dem eine möglichst gleichmäßige Beaufschlagung des Reaktionsgemisches mit Sauerstoff erfolgt.

Die EP-A-0 135 718 beschreibt ein kontinuierliches Verfahren zur Oxidation von Kohlenwasserstoffen in flüssiger Phase und speziell zur Oxidation von Cyclohexan, wobei man ein sauerstoffhaltiges Gas an mehreren Stellen im Verlauf einer Reaktionszone über nach unten gerichtete Düsenöffnungen in das flüssige Reaktionsgemisch einleitet. Die Reaktionszone ist dabei in mehrere Kammern aufgeteilt, wobei sich keine zusammenhängende Gasphase ausbilden kann. Verfahrenstechnisch umgesetzt wird dies z.B. durch eine aufrecht stehende, mit Hilfe von Lochblechen in Kammern geteilte Blasensäule, durch die von unten nach oben Cyclohexan mit einem darin gelösten Kobaltkatalysator geleitet wird. Oberhalb der Lochbleche wird über Düsen das sauerstoffhaltige Gas eingespeist, wobei durch eine genau definierte Gasaustrittsgeschwindigkeit und -austrittsmenge Blasen von definierter Größe entstehen. Somit wird das flüssige Reaktionsgemisch über das Volumen der Reaktionszone im wesentlichen gleichmäßig mit molekularem Sauerstoff in Berührung gebracht und die Nachteile der DE-A-12 87 575 werden vermieden. Bei diesem Verfahren werden die flüssigen Kohlenwasserstoffe und die sauerstoffhaltigen Gase im Gleichstrom durch den Reaktor geleitet. Dieses Verfahren ist in bezug auf den hohen Cyclohexangehalt im Abgas und die Selektivität der Oxidation bezüglich der Bildung von Cyclohexanol und Cyclohexanon noch verbesserungswürdig. Insbesondere gilt dies für eine Verringerung der säurehaltigen Nebenprodukte (z.B. Capronsäure), die durch aufwendiges Waschen mit Wasser und Natronlauge aus dem Reaktionsgemisch entfernt werden müssen, wobei Abwässer mit einer hohen Salzfracht und einem erhöhten TOC-Gehalt (total organic compounds) anfallen.

Der vorliegenden Erfindung liegt daher die Aufgabe zugrunde, ein verbessertes Verfahren zur Herstellung von Oxidationsprodukten des Cyclohexans zur Verfügung zu stellen, wobei die zuvor beschriebenen Nachteile vermieden werden.

Überraschenderweise wurde nun gefunden, daß die Aufgabe durch ein Verfahren zur Herstellung von Oxidationsprodukten des Cyclohexans durch Oxidation mit sauerstoffhaltigen Gasen in flüssiger Phase gelöst wird, wenn man flüssiges Cyclohexan und die sauerstoffhaltigen Gase im Gegenstrom durch eine Reaktionszone leitet und eine gleichmäßige Beaufschlagung des Reaktionsmediums mit Sauerstoff sicherstellt.

Gegenstand der Erfindung ist somit ein Verfahren zur Herstellung von Oxidationsprodukten des Cyclohexans durch katalytische Oxidation mit sauerstoffhaltigen Gasen in flüssiger Phase, wobei man die Gase in mindestens einer Reaktionszone im wesentlichen i gleichmäßig mit dem flüssigen Cycloheaxan in Kontakt bringt, das dadurch gekennzeichnet ist, daß man flüssiges Cyclohexan und die sauerstoffhaltigen Gase im Gegenstrom durch die Reaktionszone leitet.

Für das erfindungsgemäße Verfahren eignen sich Reaktorbehälter mit liegenden oder vorzugsweise stehenden Reaktionszonen. Nach einer bevorzugten Ausführungsform sind diese Reaktionszonen gekammert, d.h. in Abschnitte unterteilt, um eine Rückvermischung zu vermeiden. Bei liegenden Reaktionszonen kann dies z.B. durch Überläufe oder Trennwände mit Durchlässen erreicht werden; bei stehenden Reaktionszonen kann dies z.B. durch in regelmäßigen Abständen eingebaute Lochbleche bewirkt werden.

Vorteilhafterweise bildet sich durch diese apparativen Maßnahmen in der Reaktionszone keine zusammenhängende Gasphase mehr aus.

Bei dem erfindungsgemäßen Verfahren werden im Gegensatz zur EP-A-0 135 718 das flüssige Cyclohexan und die sauerstoffhaltigen Gase im Gegenstrom durch die Reaktionszone geleitet.

Zur Oxidation werden sauerstoffhaltige Gase verwendet, die molekularen Sauerstoff enthalten. Dabei liegt die Sauerstoffkonzentration vorzugsweise in einem Bereich von 5 bis 30 Vol.-%. Die sauerstoffhaltigen Gase werden vorzugsweise an mehreren Stellen im Verlauf der Reaktionszone über Düsen in das flüssige Cyclohexan eingeleitet. Zweckmäßigerweise sind dabei die Düsenöffnungen nach unten gerichtet.

Nach einer bevorzugten Ausführungsform des erfindungsgemäßen Verfahrens beträgt die Austrittsgeschwindigkeit der sauerstoffhaltigen Gase an jeder Düsenöffnung 0,01 bis 1 m/s, bevorzugt 0,03 bis 0,3 m/s.

Weiterhin bevorzugt beträgt die Austrittsmenge an jeder Düsenöffnung 0,01 bis 10 l/s, bevorzugt 0,1 bis 1,0 l/s.

Die Düsenöffnungen sind über das Volumen der Reaktionszone im wesentlich gleichmäßig verteilt. Dies erzielt man beispielsweise dadurch, daß man an mehreren Stellen in im wesentlichen gleichen Abständen im Verlauf der Reaktionszone Düsenöffnungen anordnet, die jeweils über den Querschnitt der Reaktionszone gleichmäßig verteilt sind.

Vorzugsweise entsprechen die Abstände im Verlauf der Reaktionszone dem 0,1- bis 3-fachen des Durchmessers der Reaktionszone und sind insbesondere so gewählt, daß in den aufsteigenden Gasblasen aus der vorherigen Zufuhrstelle der molekulare Sauerstoff noch nicht vollständig verbraucht ist, sondern z.B. 60 bis 90 % des ursprünglichen Gehalts beträgt. Durch diese Verfahrensmaßnahme wird eine räumlich im wesentlichen gleichmäßige Beaufschlagung des zu oxidierenden Cyclohexans mit dem sauerstoffhaltigen Gas erzielt. Dabei wird über jede Düsenöffnung im wesentlichen die gleiche Gasmenge zugeführt. Durch Kombination der beiden zuvor beschriebenen Maßnahmen, nämlich die Einspeisung im wesentlichen gleicher Gasmengen und dies an Stellen der Reaktionszone, an denen der Sauerstoff von einer vorherigen Einspeisung noch nicht vollständig abreagiert hat, führt zu einer gleichmäßigen Sauerstoffkonzentration im Reaktor. Dies unterscheidet das erfindungsgemäße Verfahren von in der DE-A-12 87 575 beschriebenen und dazu analogen Verfahren.

Durch die zuvor beschriebene Einspeisung des sauerstoffhaltigen Gases über Düsenöffnungen in die flüssige Cyclohexanphase werden Blasen mit einem definierten Durchmesser, bevorzugt von ≥ 10 mm, wie z.B. von 10 bis 100 mm, erzeugt. Diese Blasen weisen zunächst einen grösseren Durchmesser als die Gleichgewichtsblasen auf, in die sie im Verlauf der Reaktionszone zerfallen. Unter Gleichgewichtsblasen versteht man solche Blasen, die sich in einer gewissen Entfernung von der Düsenöffnung durch Zerteilungs- oder Koaleszenzvorgänge bilden, wobei sich z.B. für das System Cyclohexan/Luft Gleichgewichtsblasen mit einem mittleren Durchmesser von etwa 1 bis 10 mm ergeben.

Nach einer geeigneten apparativen Ausführung zur Einspeisung des sauerstoffhaltigen Gases kann man z.B. über Zufuhrleitungen mit einer großen Anzahl sehr kleiner Bohrungen, die einen definierten Druckverlust bewirken, jede Gasaustrittsstelle gleichmäßig mit dem sauerstoffhaltigen Gas versorgen. Aus jeder Bohrung gelangt das Gas in einen oben geschlossenen und nach unten offenen Raum, der so dimensioniert ist, daß die zuvor als bevorzugt beschriebenen Gasaustrittsmengen und -austrittsgeschwindigkeiten in das flüssige Reaktionsmedium erzielt werden. Dazu kann man z.B. durch eine dünne Bohrung die sauerstoffhaltigen Gase in eine nach unten hin offene und mit Ausnahme der dünnen Bohrung nach oben hin geschlossene Erweiterung einleiten. Die Ausführung dieser Erweiterung kann zylindrisch, kegelförmig, rechteckig, quadratisch, trompetenförmig oder glockenförmig gestaltet sein, wobei der untere Rand der Erweiterung gewünschtenfalls gezackt oder schräg sein kann. Die geometrischen Dimensionen dieser Erweiterung richten sich nach den zuvor beschriebenen Gasaustrittsgeschwindigkeiten und Gasaustrittsmengen an der Düsenöffnung und können von einem Fachmann mit den angegebenen Daten leicht berechnet werden.

Dem zu oxidierenden Cyclohexan wird ein löslicher Katalysator, bevorzugt auf Kobaltbasis, zugesetzt. Geeignete Katalysatoren sind z.B. in der DE-C-25 15 419 beschrieben.

Die Reaktionstemperatur in der Reaktionszone beträgt 120 bis 180°C, bevorzugt 130 bis 160°C und der Reaktionsdruck beträgt 5 bis 30 bar, bevorzugt 10 bis 20 bar. Druck und Temperatur werden so aufeinander abgestimmt, daß zu jedem Zeitpunkt die Umsetzung in flüssiger Phase erfolgt.

Nach einer speziellen Ausführungsform des erfindungsgemäßen Verfahrens zur Herstellung von Oxidationsprodukten des Cyclohexans wird eine stehende Reaktionszone verwendet. Dies kann verfahrenstechnisch z.B. in einem oder in mehreren in Reihe angeordneten Turmreaktor(en) umgesetzt werden. Die stehende Reaktionszone ist durch Lochbleche, die in gleichmäßigen Abständen angeordnet sind, in Kammern unterteilt. Vorteilhafterweise haben diese Lochbleche einen freien Querschnitt (Gesamtfläche der Löcher) von 3 bis 20 %, insbesondere 5 bis 10 %. Oberhalb eines jeden Lochblechs sind über den Querschnitt gleichmäßig verteilt Düsen angeordnet, wobei die Zuführungsöffnungen mit Erweiterungen versehen sein können, deren Öffnungen bevorzugt nach unten zeigen. Durch diese Reaktionszone leitet man von oben nach unten flüssiges Cyclohexan. Gleichzeitig wird über die Düsen ein sauerstoffhaltiges Gas eingespeist. Flüssiges Cyclohexan und die sauerstoffhaltigen Gase werden im Gegenstrom durch die Reaktionszone geleitet und das Reaktionsabgas wird am Kopf der Reaktionszone abgetrennt. Die Umsetzung erfolgt dabei z.B. bei einer Temperatur von etwa 140 bis 160°C und einem Druck von etwa 12 bis 16 bar.

Die Mengenverhältnisse von zugeführten, molekularen Sauerstoff enthaltenden Gasen und Cyclohexan werden vorzugsweise so aufeinander abgestimmt, daß das aus der Reaktionszone austretende Abgas einen Gehalt an molekularem Sauerstoff von nicht mehr als 2,5 Vol.-%, z.B. 0,1 bis 1,5 Vol.-% aufweist.

Das über Kopf abgetrennte Abgas weist weiterhin noch einen Cyclohexangehalt von höchstens 45 Vol.-%, bevorzugt höchstens 40 Vol.-% auf, welches nach Kondensation in den Reaktor zurückgeführt werden kann. Somit ermöglicht das erfindungsgemäße Verfahren vorteilhafterweise eine Absenkung des Cyclohexangehaltes im Abgas, was bei den verwendeten abgasseitig limitierten Reaktoren eine Erhöhung der Kapazität, d.h. des maximal zu erzielenden Cyclohexanumsatzes erlaubt. Dabei ist die Temperatur des Abgases vorzugsweise geringer als die kleinste Reaktionstemperatur in der Reaktionszone.

Die Aufarbeitung des Rohoxidats erfolgt nach den üblichen Methoden. Dazu zählen z.B. das Waschen mit Wasser und/oder wässriger Lauge, wie z.B. NaOH, wobei säurehaltige und/oder salzhaltige Abwässer entstehen. Die Auftrennung des gereinigten Oxidats in die Hauptprodukte Cyclohexanol und Cyclohexanon sowie die Abtrennung von nicht umgesetztem Cyclohexan und von in untergeordnetem Maße entstehenden weiteren Oxidationsprodukten erfolgt in üblicher Weise z.B. durch fraktionierte Destillation.

Das erfindungsgemäße Verfahren ermöglicht vorteilhafterweise eine gleichmäßige Verteilung des molekularen Sauerstoffs im zu oxidierenden Cyclohexan, wobei es keiner weiteren mechanischen Mittel zur Durchmischung bedarf. Insbesondere wird keine zusammenhängende Gasphase ausgebildet.

Durch die Führung der Reaktanten im Gegenstrom durch die Reaktionszone kann man die zuvor beschriebenen Nachteile vermeiden. So wird der Gesamtumsatz des Cyclohexans erhöht und gleichzeitig die Selektivität der Oxidation gegenüber einer bevorzugten Bildung von Cyclohexanol und Cyclohexanon verbessert. Insbesondere durch eine Verringerung des Gehaltes an Carbonsäuren (Capronsäure) bei den Oxidationsprodukten kommt es zu einer Entlastung der Abwässer, die beim Mschen des Rohoxidats mit Wasser und wässriger Lauge anfallen.

Das Verfahren wird anhand der folgenden, nicht einschränkenden Beispiele erläutert.

### Beispiele

### Beispiel 1 (Vergleich)

In einem Oxidationssystem, bestehend aus drei nacheinander geschalteten Reaktoren (Volumen jeweils 40 m³, Höhe 16 m, Durchmesser 1,8 m), einem Aufarbeitungsteil (Entfernung der Säuren durch Wasserwäsche und Extraktion sowie Neutralisation mit Natronlauge) sowie einem Destillationsteil wird Cyclohexan oxidiert. Die Reaktoren sind mit einem Gasverteiler versehen, der die Luft gleichmäßig über den Reaktorquerschnitt und die Reaktorhöhe verteilt. Der Flüssigkeitsinhalt wird in 7 Ebenen in einem Abstand von 2 m begast. Zur Verringerung der Rückvermischung sind im Reaktor jeweils Lochbleche (Lochdurchmesser: 40 mm, auf den Reaktorquerschnitt bezogene freien Fläche: 4 %) 30 cm unterhalb der Gasverteiler eingebaut. Der Gasverteiler jeder Ebene ist derart gestaltet, daß die Luft durch 33 Bohrungen (Ø 3 mm), die gleichmäßig über den Reaktorquerschnitt verteilt sind, an der Unterseite der Begasungsrohre (NW 32) austritt. Jede Bohrung ist mit einem Hüllrohr (L 60 mm, Ø 25 mm) versehen. Die Gasaustrittsgeschwindigkeit unter Betriebsbedingungen beträgt 0,25 m/s. Das Reaktorsystem (Kopfdruck ca 13 bar) wird von unten mit 80 t/h Cyclohexan bei einer Temperatur von 140°C beaufschlagt und im Gleichstrom betrieben. Das Cyclohexan wird vor Eintritt in den ersten Reaktor mit 0,1 ppm Co in Form des Ethylhexensäuresalzes versetzt.

### Beispiel 2 (erfindungsgemäß)

Das Oxidationssystem wird analog zu Beispiel 1, jedoch im Gegenstrom betrieben, wobei die Zugabe des Cyclohexans von oben erfolgt.

Die Ergebnisse der Versuche sind in Tabelle 1 zusammengefasst.

**Tabelle 1**

| | Beispiel 1 (Gleichstrom) | Beispiel 2 (Gegenstrom) |
|---|---|---|
| max. Cyclohexanumsatz [%] (abgasseitig limitiert) | 4,9 | 5,6 |
| Cyclohexanumsatz [%] | 4,9 | 5,0 |
| Selektivität bzgl. Cyclohexanol/Cyclohexanon [%] | 77,5 | 78,3 |
| Abgastemperatur [°C] | 153 | 141 |

Wie die Tabelle 1 belegt, wird die Cyclohexanoxidation durch das Gegenstromverfahren deutlich verbessert, wobei noch Reserven in bezug auf eine weitere Kapazitätserhöhung (abgasseitig limitierter maximaler Cyclohexanumsatz) vorhanden sind.

## Patentansprüche

1. Verfahren zur Herstellung von Oxidationsprodukten des Cyclohexans durch katalytische Oxidation mit sauerstoffhaltigen Gasen in flüssiger Phase bei erhöhter Temperatur, wobei man die Gase in mindestens einer stehenden Reaktionszone im wesentlichen gleichmäßig mit dem flüssigen Cyclohexan im Gegenstrom in Kontakt bringt, **dadurch gekennzeichnet, daß** das Einleiten der Gase in die Reaktionszone über mehrere Düsen erfolgt und die Reaktionszone durch Lochbleche in mehrere Kammern aufgeteilt ist, so daß sich in der Reaktionszone keine zusammenhängende Gasphase ausbildet.

2. Verfahren nach Anspruch 1, **dadurch gekennzeichnet, daß** die Temperatur des Abgases niedriger als die kleinste Reaktionstemperatur in der Raktionszone ist.

3. Verfahren nach einem der Ansprüche 1 oder 2, **dadurch gekennzeichnet, daß** die Austrittsgeschwindigkeit der sauerstoffhaltigen Gase an jeder Düsenöffnung 0,01 bis 1 m/s, bevorzugt 0,03 bis 0,3 m/s, beträgt und daß die Austrittsmenge an jeder Düsenöffnung 0,001 bis 10 1/s, bevorzugt 0,1 bis 1,0 1/s, beträgt.

4. Verfahren nach einem der Ansprüche 1 bis 3, **dadurch gekennzeichnet, daß** man die sauerstoffhaltigen Gase im Verlauf der Reaktionszone in Abständen, die dem 0,1- bis 3-fachen des Durchmessers der Reaktionszone entsprechen, zuführt, wobei die zuführenden Düsenöffnungen gleichmäßig über den Querschnitt der Reaktionszone verteilt sind.

5. Verfahren nach einem der Ansprüche 1 bis 4, **dadurch gekennzeichnet, daß** die Reaktionszone in mehrere Kammern aufgeteilt ist, die miteinander in Verbindung stehen.

6. Verfahren nach einem der Ansprüche 1 bis 5, **dadurch gekennzeichnet, daß** die Einspeisung des sauerstoffhaltigen Gases in die Reaktionszone an Stellen erfolgt, an denen der Sauerstoff aus einer vorherigen Einspeisung noch nicht vollständig abreagiert hat.

7. Verfahren nach einem der Ansprüche 1 bis 6, **dadurch gekennzeichnet, daß** die Reaktionstemperatur 120 bis 180°C, bevorzugt 130 bis 160°C beträgt.

8. Verfahren nach einem der Ansprüche 1 bis 7, **dadurch gekennzeichnet, daß** der Reaktionsdruck 5 bis 30 bar, bevorzugt 10 bis 20 bar beträgt.

9. Verfahren nach einem der Ansprüche 1 bis 8, **dadurch gekennzeichnet, daß** die Sauerstoffkonzentration der sauerstoffhaltigen Gase 5 bis 30 Vol.-% beträgt.

10. Verfahren nach einem der Ansprüche 1 bis 9, **dadurch gekennzeichnet, daß** der Cyclohexangehalt im Abgas höchstens 45 Vol.-%, bevorzugt höchstens 40 Vol.-% beträgt.

## Claims

1. A process for preparing oxidation products of cyclohexane by catalytic oxidation with oxygen-containing gases in the liquid phase at elevated temperature, where the gases are brought into contact essentially uniformly with the liquid cyclohexane in countercurrent in at least one upright reaction zone, wherein the gases are introduced into the reaction zone through a plurality of nozzles and the reaction zone is divided into a plurality of chambers by means of perforated metal sheets so that no continuous gas phase is formed in the reaction zone.

2. A process as claimed in claim 1, wherein the temperature of the waste gas is lower than the lowest reaction temperature in the reaction zone.

3. A process as claimed in either of claims 1 or 2, wherein the exit velocity of the oxygen-containing gases at each nozzle orifice is from 0.01 to 1 m/s, preferably from 0.03 to 0.3 m/s, and the amount coming out of each nozzle orifice is from 0.001 to 10 l/s, preferably from 0.1 to 1.0 l/s.

4. A process as claimed in any of claims 1 to 3, wherein the oxygen-containing gases are fed in at intervals along the reaction zone which correspond to from 0.1 to 3 times the diameter of the reaction zone, with the nozzle orifices which feed in the gases being distributed uniformly over the cross section of the reaction zone.

5. A process as claimed in any of claims 1 to 4, wherein the reaction zone is divided into a plurality of chambers which are connected to one another.

6. A process as claimed in any of claims 1 to 5, wherein the oxygen-containing gas is fed into the reaction zone at points at which the oxygen from a previous feed point has not yet reacted completely.

7. A process as claimed in any of claims 1 to 6, wherein the reaction temperature is from 120 to 180°C, preferably from 130 to 160°C.

8. A process as claimed in any of claims 1 to 7, wherein the reaction pressure is from 5 to 30 bar, preferably from 10 to 20 bar.

9. A process as claimed in any of claims 1 to 8, wherein the oxygen concentration of the oxygen-containing gases is from 5 to 30 % by volume.

10. A process as claimed in any of claims 1 to 9, wherein the cyclohexane content of the waste gas is at most 45 % by volume, preferably at most 40 % by volume.

## Revendications

1. Procédé de préparation de produits d'oxydation du cyclohexane par oxydation catalytique au moyen de gaz contenant de l'oxygène, en phase liquide et à température élevée, dans lequel on met les gaz en contact avec le cyclohexane liquide, dans au moins une zone réactionnelle verticale et en contre-courant, **caractérisé en ce que** les gaz sont introduits dans la zone réactionnelle par plusieurs ajutages et que la zone réactionnelle est divisée par des tôles perforées en plusieurs chambres, de sorte qu'aucune phase gazeuse d'un seul tenant ne se forme dans la zone réactionnelle.

2. Procédé selon la revendication 1, **caractérisé en ce que** la température du gaz effluent est inférieure à la plus basse température de réaction de la zone réactionnelle.

3. Procédé selon l'une des revendications 1 ou 2, **caractérisé en ce que** la vitesse de sortie des gaz contenant de l'hydrogène à chacune des bouches des ajutages est de 0,01 à 1 m/s, de préférence de 0,03 à 0,3 m/s, et que le débit de sortie à chaque bouche d'ajutage est de 0,001 à 10 l/s, de préférence de 0,1 à 1,0 l/s.

4. Procédé selon l'une des revendications 1 à 3, **caractérisé en ce que** l'on injecte les gaz contenant de l'oxygène au long de la zone réactionnelle à intervalles correspondant à 0,1 à 3 fois le diamètre de la zone réactionnelle, et que les bouches d'injection sont uniformément réparties sur la section transversale de la zone réactionnelle.

5. Procédé selon l'une des revendications 1 à 4, **caractérisé en ce que** la zone réactionnelle est divisée en plusieurs zones communicantes.

6. Procédé selon l'une des revendications 1 à 5, **caractérisé en ce que** les gaz contenant de l'oxygène sont injectés dans la zone réactionnelle en des points où l'oxygène provenant d'une alimentation antérieure n'avait pas encore totalement réagi.

7. Procédé selon l'une des revendications 1 à 6, **caractérisé en ce que** la température de réaction est de 120 à 180°C, de préférence de 130 à 160°C.

8. Procédé selon l'une des revendications 1 à 7, **caractérisé en ce que** la pression de réaction est de 5 à 30 bar, de préférence de 10 à 20 bar.

9. Procédé selon l'une des revendications 1 à 8, **caractérisé en ce que** la concentration en oxygène des gaz contenant de l'oxygène est de 5 à 30% en volume.

10. Procédé selon l'une des revendications 1 à 9, **caractérisé en ce que** la teneur en cyclohexane du gaz effluent est au maximum de 45% en volume , de préférence au maximum de 40% en volume.
